# EUROPÄISCHE PATENTSCHRIFT

(11) **EP 1 376 136 B1**
(45) Veröffentlichungstag und Bekanntmachung des Hinweises auf die Patenterteilung: **15.03.2006**
(21) Anmeldenummer: 03012914.2
(22) Anmeldetag: 06.06.2003
(51) Int. Cl.: G01N 33/74

(54) **Verfahren zur Testung des hormonellen Effekts von Substanzen**
Assay for the determination of the hormonal effect of compounds
Méthode de détermination de l'effet hormonal de molécules

(30) Priorität: 12.06.2002 DE 10226674
(43) Veröffentlichungstag der Anmeldung: 02.01.2004
(73) Patentinhaber: Schering Aktiengesellschaft, 13353 Berlin (DE)
(72) Erfinder: Wolf, Siegmund, Dr., 07745 Jena (DE); Meyer, Rene, 08468 Reichenbach (DE); Obendorf, Maik, Dr., 99423 Weimar (DE); Schröder, Jens, Dr., 07743 Jena (DE)

(56) Entgegenhaltungen:
- EP-A- 1 255 113
- MURPHY DEREK B ET AL: "Human brain factor 1, a new member of the fork head gene family" GENOMICS, Bd. 21, Nr. 3, 1994, Seiten 551-557, XP002258156 ISSN: 0888-7543
- DATABASE GENBANK [Online] NCBI; 13. Mai 2002 (2002-05-13) "Homo sapiens forkhead box G1C (FOXG1C), mRNA" Database accession no. XM007233 XP002258157
- MCKENNA N ET AL: "Nuclear receptor coregulators: Cellular and molecular biology" ENDOCRINE REVIEWS, BALTIMORE, MD, US, Bd. 20, Nr. 3, Juni 1999 (1999-06), Seiten 321-344, XP002234409
- BEATO M ET AL: "Steroid hormone receptors: An update" HUMAN REPRODUCTION UPDATE, Bd. 6, Nr. 3, Mai 2000 (2000-05), Seiten 225-236, XP009019093 ISSN: 1355-4786

## Beschreibung

Die Erfindung bezieht sich auf ein Verfahren zur Testung des hormonellen Effekts von Substanzen und auf ein Verfahren zur Bestimmung von Störungen im Co-Modulationsmechanismus von Androgenrezeptoren den Co-Aktivator Forkhead Box G1C (FOXG1C).

Bei der Beurteilung von Substanzen für eine mögliche pharmazeutische Anwendung ist es allgemein üblich, diese Substanzen auf eine eventuelle hormonelle Wirkung, insbesondere auf eine möglicherweise vorhandene androgene oder antiandrogene Aktivität, zu prüfen. Bei der Verabreichung pharmakologisch wirksamer Substanzen sind Kenntnisse über hormonelle Effekte, insbesondere androgene oder antiandrogene Effekte, dieser Substanzen in manchen Fällen von Bedeutung, da sie beim Patienten unerwünschte Nebenwirkungen hervorrufen können. Zur Prüfung der hormonellen Wirkung von Substanzen kommen insbesondere Verfahren zum Einsatz, bei denen die Fähigkeit der Substanzen gemessen wird, an die Hormonrezeptoren zu binden und deren Transkriptionsaktivität zu aktivieren.

Kenntnisse über hormonelle Effekte von Substanzen sind aber nicht nur bei potentiellen Pharmaka von Interesse, sondern auch bei nicht-pharmazeutischen Substanzen, da von vielen, in der Umwelt vorhandenen Substanzen angenommen wird, daß sie bei Teilen der Bevölkerung eine androgene oder antiandrogene bzw. estrogene oder antiestrogene Aktivität aufweisen können. Möglicherweise wird dadurch eine unerwünschte, schädliche Wirkung hervorgerufen.

Es besteht also ein ganz erhebliches Bedürfnis für ein Verfahren mit dem in zuverlässiger, empfindlicher, einfacher, kostengünstiger und schneller Weise eine Aussage über den hormonellen Effekt von Substanzen getroffen werden kann. Die bisher bekannten Verfahren genügen dem nicht.

Der vorliegenden Erfindung liegt deshalb die Aufgabe zu Grunde, ein Verfahren bereitzustellen, mit dem in zuverlässiger, empfindlicher, einfacher, kostengünstiger und schneller Weise eine Aussage über den hormonellen Effekt der zu testenden Substanzen getroffen werden kann.

Erfindungsgemäß wird dies in überraschender Weise erreicht durch ein Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon, insbesondere ein humanes nukleäres Rezeptor-Protein oder ein Fragment davon, kodiert, und der andere Vektor DNA enthält, die für den Co-Modulator FOXG1C oder ein Fragment davon kodiert, der Substanz ausgesetzt werden und
b) die Transkriptionsaktivität, die der nukleäre Rezeptor oder dessen Fragment in Anwesenheit des Co-Modulators oder dessen Fragment auslöst, und/oder der Einfluß der Substanz auf die Interaktion zwischen dem Rezeptor oder dessen Fragment und dem Co-Modulator oder dessen Fragment durch Protein-Protein-Interaktion oder Protein-Protein-DNA-Interaktion gemessen wird.

Es wurde überraschenderweise gefunden, daß mit dem erfindungsgemäßen Verfahren in zuverlässiger, empfindlicher, einfacher, schneller und kostengünstiger Weise getestet werden kann, ob Substanzen, die beispielsweise umwellrelevant oder pharmakologisch von Interesse sein können, einen hormonellen Effekt, insbesondere einen androgenen oder antiandrogenen Effekt, ausüben.

Im erfindungsgemäßen Verfahren werden mit einem Vektor transformierte Zellen eingesetzt, wobei die Vektoren DNA aufweisen, die für ein nukleäres Rezeptor-Protein oder ein Fragment davon kodiert.

Die Superfamilie der nukleären Rezeptoren (NRs), zu der mehr als 50 verschiedene Proteine gehören, ist eine Gruppe verwandter Transkriptionsfaktoren, die die Transkription des jeweiligen Zielgens als Reaktion auf spezifische Liganden, z. B. Hormone, steuern. Die Familie kann nach bestimmten Charakteristika, wie z.B. Dimerisationsstatus, Art des Liganden oder Struktur des DNA-Reaktionselements, in mehrere Subfamilien unterteilt werden (Beato et al., 2000, Human Reproduct. Update, 6, 225-236). Charakteristisches Merkmal der NRs ist die übereinstimmende Struktur der funktionellen Domäne (mit den Bezeichnungen A bis F) mit einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Reaktionselementen verantwortlich ist und aus zwei Zinkfinger-Motiven besteht, einer variablen Scharnierdomäne und einer konservierten multifunktionalen C-terminalen Ligandenbindungsdomäne (LBD) mit Dimerisations- und Liganden-abhängiger Transaktivierungsfunktion (AF-2). Im Anschluß daran folgt die am weitesten C-terminal gelegene Region, deren Funktion nicht bekannt ist und die bei Rezeptoren wie z.B. PR (ProgesteronRezeptor), PPAR (Peroxisomproliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige NRs (z.B. den Androgen-Rezeptor (AR)) wurde nachgewiesen, daß die N-terminale Region in der Lage ist, mit der C-terminalen Region zu interagieren (Brinkmann et al., 1999, J. Steroid Biochem. and Mol. Biol., 69, 307-313). Steroidhormonrezeptoren wie z.B. Estrogen- (ER), Progesteron- (PR), Glukokortikoid- (GR), Mineralokortikoid- (MR) und Androgenrezeptoren (AR) binden steroidale Liganden, die sich von Pregnenolon ableiten, wie die Progestine, die Estrogene, die Glukokortikoide und die Mineralokortikoide, sowie Androgene. Die Ligandenbindung aktiviert den Rezeptor und steuert die Expression entsprechender Zielgene.

Wie vorstehend ausgeführt wurde, werden in Schritt a) des erfindungsgemäßen Verfahrens Zellen verwendet, die ferner einen Vektor enthatten, der für den Co-Modulator FOXG1C oder ein Fragment davon kodierende DNA enthält

Die sog. Co-Modulatoren sind eine Klasse von Proteinen, die bei der Aktivierung (Co-Aktivatoren) bzw. Repression (Co-Repressoren) der Gentranskription als Brückenmoleküle zwischen dem Transkriptionsinitiationskomplex und den NRs dienen (McKenna et al., 1999, Endocr. Rev., 20, 321-347). Ein Co-Aktivator muß fähig sein, die Rezeptorfunktion zu verstärken und in Anwesenheit eines Agonisten mit der Aktivierungsdomäne von NRs direkt zu interagieren. Er muß auch mit dem basalen Transkriptionsapparat interagieren, und schließlich darf er nicht selbst die basale Transkriptionsaktivität verstärken. Die meisten Co-Modulatoren interagieren mit Hilfe eines oder mehrerer LXXLL-Motiv(en) (NR-Boxes) mit der AF-2-Domäne von NRs, jedoch wurden auch einige Co-Modulatoren beschrieben, die mit anderen NR-Regionen interagieren (Ding et al., 1998. Mol. Endocrinol., 12, 302-313). Ferner wurden viele Co-Modulatoren identifiziert, die in ähnlicher Weise mit mehreren verschiedenen NRs interagieren, so daß günstigerweise der Spezifizitätsgrad jedes Co-Modulators getestet werden sollte.

Im erfindungsgemäßen Verfahren wird der mit FOXG1C bezeichnete Co-Modulator oder das die Aminosäuren 175 bis 489 aufweisende Fragment von FOXG1C eingesetzt. Die cDNA-Sequenz wurde beschieben (Genbank XM_007233) und kodiert für 489 Aminosäuren (Li & Vogl, 1993, Proc. Natl. Acad. Sci. U.S.A., 90, 4490-4494). Ein DH5 alpha E. coli-Klon, der ein für die Aminosäure 175-489 von FOXG1C kodierende Plasmid enthält, wurde unter der Nummer DSM 15043 am 5. Juni 2002 bei der Deutschen Sammlung für Mikroorganismen und Zellkulturen hintertegt.

Bei Verwendung dieser Proteine kann in besonders zuverlässiger, empfindlicher, einfacher, kostengünstiger und schneller Weise das erfindungsgemäße Verfahren durchgeführt werden. Ferner weisen die FOXG1C-Fragmente, insbesondere das die Aminosäuren 175 bis 489 aufweisende Fragment von FOXG1C, den Vorteil auf, daß sie leichter handhabbar und klonierbar sind, aber noch die fünktiellen Eigenschaften von FOXG1C aufweisen.

Bei FOXG1C handelt es sich um einen Co-Aktivator für den humanen Androgenrezeptor und andere nukleäre Rezeptoren, der die Interaktion zwischen einem Androgen und dem Rezeptor verstärkt. Die Sequenz von FOXG1C ist bereits in der Genbank XM_007233 beschrieben; allerdings ist dort keine Interaktion mit nukleären Rezeptoren, insbesondere dem AR, angegeben. Die vorliegende Erfindung beruht auf der überraschenden Erkenntnis, daß eine Interaktion zwischen nukleären Rezeptoren, insbesondere AR, einerseits und FOXG1C andererseits vorliegt sowie eine Verstärkung der AR-vermittelten Transaktivierung festgestellt werden konnte. FOXG1C ist ein Protein, das als Co-Mediator fungiert, indem es nach Bindung von Steroiden an den nukleären Rezeptor den Transkriptionseffekt verstärkt oder reprimiert und darüber hinaus die Bindung und Aktivierung des nukleären Rezeptors an Moleküle fördert, denen früher keine hofmonefte Wirkung zugeschrieben wurde.

Das Protein FOXG1C stellt einen Co-Aktivator für den Androgenrezeptor und weitere nukleäre Rezeptoren dar, wie Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren; im erfindungsgemäßen Verfahren werden diese Rezeptoren bevorzugt eingesetzt, da damit die vorstehenden Vorteile des erfindungsgemäßen Verfahrens besonders günstig erreicht werden können.

In erfindungsgemäßen Verfahren können auch Vektoren, die für Fragmente vorstehender Proteine kodieren, eingesetzt werden. Unter dem Ausdruck "Fragmente- im Zusammenhang mit vorstehenden Proteinen werden solche verstanden, die eine Aminosäure oder mehrere Aminosäuren weniger als die Proteine in voller Länge und noch die funktionellen Eigenschaften eines nukleären Rezeptors oder eines Co-Modulators aufweisen.

Wie bereits vorstehend ausgeführt wurde, werden im erfindungsgemäßen Verfahren in Schritt a) Zellen eingesetzt, die mit zwei Vektoren transfektiert sind, die für spezielle Proteine kodierende DNA enthalten. Diese Zellen sind also in der Lage, diese beiden unterschiedlichen Proteine zu exprimieren.

Vorzugsweise sind die Zellen etablierte Zelllinien und/oder eukaryotische Zellen, insbesondere Prostatazellen, Nervenzellen, Gliazellen, Fibroblasten, Blutzellen, Osteoblasten, Osteoklasten, Hepatozyten, Epithelzellen oder Muskelzellen. Mit den etablierten Zelllinien kann das erfindungsgemäße Verfahren besonders kostengünstig und schnell durchgeführt werden. Bei Verwendung eukaryotischer Zellen, insbesondere vorstehend aufgeführter eukaryotischer Zellen, können mit dem erfindungsgemäßen Verfahren vorteilhafterweise besonders aussagekräftige Ergebnisse erhalten, werden.

In einer bevorzugten Ausführungsform des erfindungsgemäßen Verfahrens werden eukaryotische Expressionsvektoren eingesetzt, z.B. pCMX oder pSG5. Bei Verwendung dieser Vektoren, insbesondere in Verbindung mit vorstehenden etablierten Zelllinien und/oder eukaryotischen Zellen, kann das erfindungsgemäße Verfahren besonders günstig und schnell durchgeführt werden, und es werden besonders aussagekräftige Ergebnisse erhalten.

Der Fachmann kennt Verfahren und dazu notwendige Materialien, um die für die vorstehenden Proteine kodierende DNA in einen Vektor zu inserieren, diesen dann in die Zellen einzubringen und die so erhaltenen Zellen unter geeigneten Kulturbedingungen zu kultivieren, damit sie diese Proteine exprimieren können.

Gemäß Schritt b) des erfindungsgemäßen Verfahrens kann die Transkriptionsaktivität gemessen werden, die der nukleäre Rezeptor oder dessen Fragment in Anwesenheit des Co-Modulators oder dessen Fragment auslöst. Dies kann beispielsweise durch Detektion eines Reportergens erfolgen.

Reportergene sind Gene oder Genfragmente, die mit anderen Genen oder regulatorischen Sequenzen gekoppelt werden, um die Aktivität dieser Sequenzen nachweisbar zu machen. Reportergene erzeugen Genprodukte, die möglichst einfach nachweisbar sind, z. B. photometrisch durch Farbreaktionen. Häufig verwendete Reportergene sind das Gen für β-Galactosidase, das Gen für die alkalische Phosphatase, das Gen für Chloramphenicol-Acetyltransferase, das Gen für die Catechol-Dioxygenase, das Gen für das "green fluorescent protein" sowie verschiedene Luciferase-Gene, die die Zellen zum Leuchten bringen können.

Solche Reportergene können ebenfalls mittels Vektoren, insbesondere eukaryotischer Expressionsvektoren, in die Zellen eingebracht werden. Beispiel eines Vektors, der für ein Reportergen kodierende DNA enthält, ist der Vektor MMTV-Ludferase, der zur Messung der androgenen Wirkung von Substanzen eingesetzt wird.

Substanzen mit einem hormonellen Effekt, insbesondere einem androgenen/antiandrogenen Effekt, sind dann an der erhöhten bzw. verminderten Aktivität des Reportergens erkennbar.

Die Messung des Einflusses der Testsubstanz auf die Interaktion zwischen dem Rezeptor oder dessen Fragment und dem Co-Modulator oder dessen Fragment kann auch durch Bestimmung der Protein-Protein-Interaktion, z. B. durch Doppelt-Hybrid Systeme, Immunpräzipitation, GST-Pull-down-Assays, FRET-Analayse und ABCD-Assays sowie durch Bestimmung der Protein-Protein-DNA-Interaktion, wie durch Gelretardationsassays, erfolgen.

Es wurde ferner gefunden, daß FOXG1C sehr gut als Indikator androgenbedingter Erkrankungen verwendet werden kann. Relevante androgenbedingte Erkrankungen, wie z.B. Prostatakrebs, erektile Dysfunktion, Infertilität, Glatzenbildung, Akne oder Hypogonadismus, sowie Androgenresistenzsyndrome, wie z.B. die testikuläre Feminisierung, beruhen auf Defekten im Co-Modulationsmechanismus zwischen AR und FOXG1C, ebenso wie Störungen der Libido, der Gefühlslage und bei kognitiven Störungen. Eine Möglichkeit bei Patienten mit derartigen Störungen besteht somit in der Messung der relativen Konzentrationen von AR und FOXG1C, wobei diese Messung günstigerweise in Körperflüssigkeiten, Körperzellen oder Körpergewebe extrakorporal erfolgt. Dies ist möglich durch Anwendung quantitativer Verfahren zur Messung der relativen Menge beider Moleküle bei dem jeweiligen Patienten, bei denen beispielsweise Antikörper sowohl gegen AR als auch gegen FOXG1C oder Nukleinsäuresonden gegen deren mRNA eingesetzt werden können. Es gibt mehrere Verfahren zur Messung dieser komparativen Raten, die dem Fachmann bekannt sind; auch kennt er hierzu geeignete Materialien und Vorrichtungen, wie Radioimmunassay, ELISA-Test, Immunfärbung, RT-PCR, Western-Blot, Northern-Blot, DNA-Chip oder Protein-Chip. Darüber hinaus ist es möglich, mit Hilfe der FOXG1C-cDNA in üblicher Weise Sonden für ein PCR-Assay zu konstruieren, mit dem sich bei bestimmten Patienten Mutationen der normalen DNA-Sequenz nachweisen oder Transkripte für den Northern Blot Assay bzw. eine DNA für In-situ-Hybridisierungsassays generieren lassen.

Das gemessene Verhältnis von AR zu FOXG1C kann dabei größer oder kleiner als das bei Gesunden sein. Der bei Gesunden vorliegende Normalwert kann in einfacher Weise beispielsweise dadurch bestimmt werden, daß das Verhältnis von AR zu FOXG1C bei einer Vielzahl von gesunden Probanden gemittelt wird. Durch den Vergleich des Normalwertes mit dem ermittelten Verhältnis von AR zu FOXG1C bei dem zu untersuchenden Patienten kann festgestellt werden, ob der Wert für das ermittelte Verhältnis größer oder kleiner als der Normalwert ist.

Da die Konzentration von FOXG1C und/oder AR im Gewebe unterschiedlich sein kann, z.B. ist die Konzentration von FOXG1C im Gehirn und Hoden sehr groß, wohingegen sie in anderen Organe, wie Leber, Herz, Thymus und Prostata sehr gering ist, sind die unterschiedlichen Gewebekonzentrationen für die Auswertung zu berücksichtigen, d.h. der Testwert und der Normalwert sollen vom gleichen Gewebe stammen. Da bei der Konzentration auch Spezienunterschiede vorliegen können, sind diese bei der Auswertung auch zu berücksichtigen.

Eine andere Möglichkeit für Bestimmung von Defekten im Co-Modulationsmechanismus zwischen AR und FOXG1C kann darin bestehen, nur die Konzentration von FOXG1C zu messen, wobei man dabei davon ausgeht, daß die FOXG1C-Konzentration zumindest annähernd konstant ist. Ist hierbei eine geringere als die normale FOXG1C-Konzentration gemessen worden, heißt dies, daß sich das Verhältnis von AR zu FOXG1C verschoben hat, was wiederum als Hinweis auf eine Störung im Co-Modulationsmechanismus hinweist.

Es ist also möglich, mit einer FOXG1C spezifischen Sonde Änderungen in der FOXG1C-Expression und damit im Verhältnis zu AR zu bestimmen. Solche Änderungen können bei verschiedenen Krankheitsbildern ursächlich involviert sein oder als Folgeerscheinung auftreten.

Derartigen Messungen des AR/FOXG1C-Verhältnisses liegt die überraschende und auf die Auffindung und Charakterisierung von FOXG1C beruhende Erkenntnis zugrunde, daß beispielsweise ein Androgenresistenzsyndrom auf einer Störung des Gleichgewichts zwischen AR- und FOXG1C-Prävatenz in den Zielzellen beruhen kann. Zuviel FOXG1C könnte zu einer Überempfindlichkeit des AR-Systems führen, so daß es auf Moleküle reagiert, die normalerweise keinen androgenen Effekt haben. Umgekehrt führt das Fehlen oder eine Fehlfunktion von FOXG1C auf allen Ebenen zur Androgenresistenz. Der Nachweis von zu viel FOXG1C bei einem Patienten spräche für die Anwendung von Mitteln zur Down-Regulation, wie z.B. Antisense- oder ähnlichen Medikamenten, um unter klinischen Bedingungen den FOXG1C-Titer bei dem jeweiligen Patienten zu reduzieren. Dasselbe kann durch Moleküle erreicht werden, die in der Lage sind, die Interaktion zwischen AR und FOXG1C zu hemmen. Hat ein Patient zu wenig FOXG1C, kann man ihm FOXG1C-cDNA, -Protein oder -DNA über verschiedene an sich bekannte Mechanismen zuführen, um auf diese Weise den Titer des aktiven FOXG1C zu erhöhen. Möglich ist auch eine Anhebung der Konzentration oder der Aktivität von FOXG1C durch niedrigmolekulare Arzneimittel oder durch Stimulation der Eigensynthese mit Hilfe spezifischer FOXG1C-Promoter-Proteine.

Die Erfindung wird unter Bezugnahme auf die folgenden Abbildungen näher erläutert, wobei
Abbildung 1 eine schematische Darstellung des Androgenrezeptors mit Kennzeichnung der von den Aminosäuren 325 bis 919 reichenden Androgenrezeptor-Domäne (AR 2) ist, die zur Interaktion mit FOXG1C in Anwesenheit von Androgen fähig ist;
Abbildung 2 die Gewebsverteilung von FOXG1C darstellt, in 2a in verschiedenen Geweben (Mensch und Nager), in 2b in verschiedenen humanen Gehimregionen und in 2c in Rattengehim von jungen und älteren Tieren; und
Abbildung 3 die Interaktion des FOXG1C oder SRC-1a mit dem Androgenrezeptor in PC3-ARwt-Zellen.

Das folgenden Beispiele illustriert die Erfindung näher, ohne sie jedoch darauf zu beschränken.

### Beispiel 1:

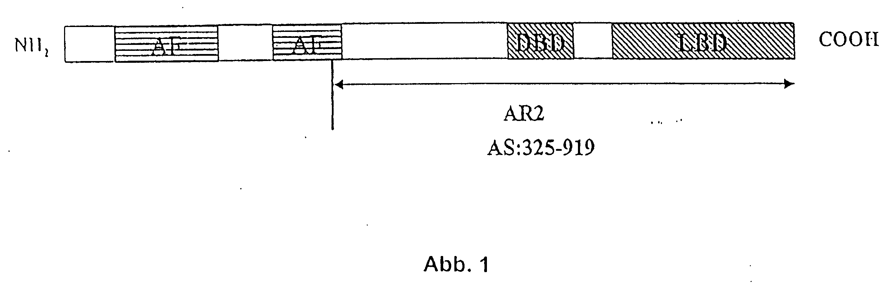

Unter Verwendung einer cDNA-Bibliothek aus fetalem Gehim (Clontech MATCHMAKER) und eines humanen AR-Fragments, das für die Aminosäuren 325 bis 919 kodiert, als Sonde (Abbildung 1) wurde ein Screening mittels eines üblichen zwei-hybrid Hefe-Systems in Anwesenheit von Androgen 10⁻⁶M DHT durchgeführt. In Übereinstimmung mit den Anweisungen des Herstellers (Clontech) betrug die Zahl der gescreenten Klone 2x10⁷. Die Zähl der unabhängigen Klone betrug laut Angaben des Herstellers 3,5x10⁶. Davon wurden 300 positive Klone ausgewählt und mit einem β-Galaktosidase-Assay getestet, wobei 40 als IacZ-positive Klone bestätigt wurden. Die Inserts dieser Klone wurden durch PCR amplifiziert. Mittels Restriktionsfragmentanalysen und Sequenzierung wurden mindestens 39 verschiedene Klone identifiziert. Einer davon war ein Klon mit einem 1553 bp umfassenden Insert (739 bp - 2292 bp), das für einen Teil des ORF (Open reading frame) von FOXG1C kodiert (Genbank-Zugangsnummer XM_007233).

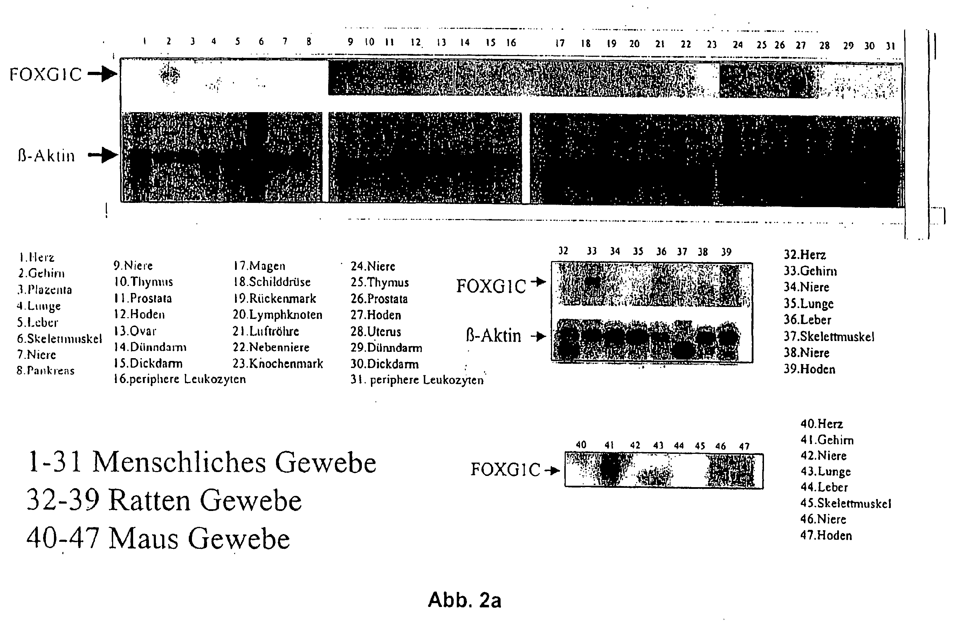

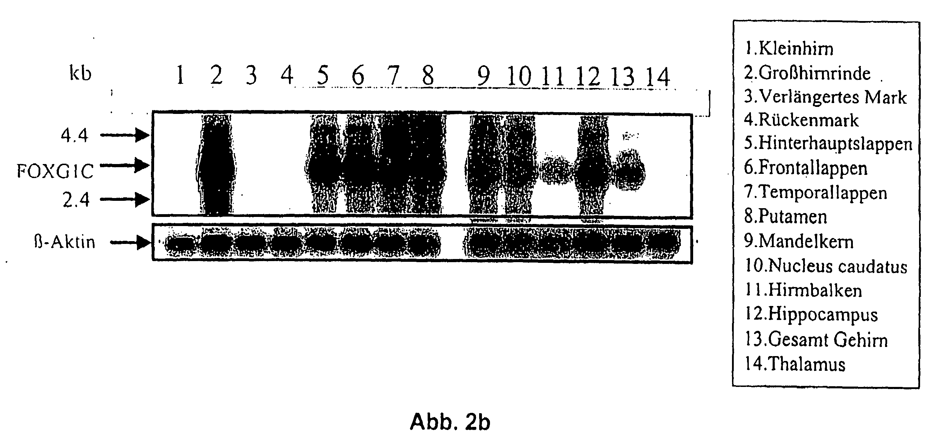

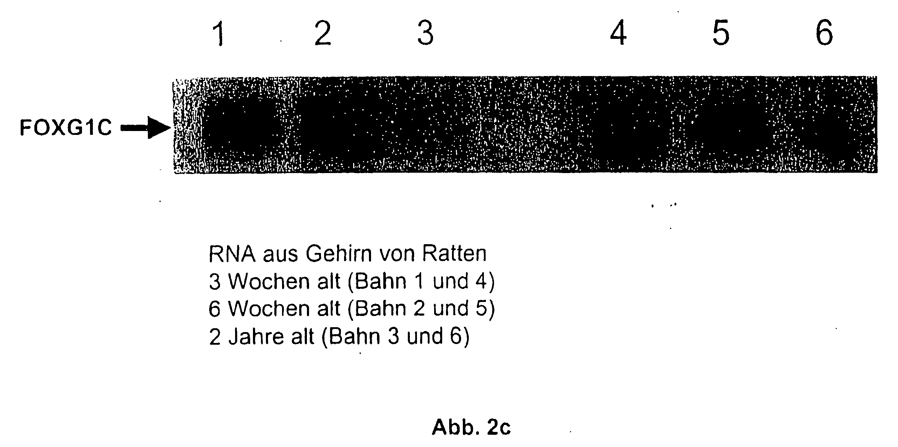

Das aus 1255 bp bestehende Fragment (963 bp - 2218 bp) der FOXG1C-cDNA Sequenz diente als Sonde für humane Northern Blots. Ein Transcript (3.2 kb) wurden in verschiedenen Geweben entdeckt (Abbildung 2). Die Northern Blots wurden gegebenenfalls mit einer β-Aktin Probe hybridisiert, um die einheitliche Ladung des Gels zu bestätigen.

Abbildung 2 zeigt die Gewebsverteilung von FOXG1C, die in an sich bekannter Weise mittels Northern-Blot-Analyse untersucht wurde. Aus verschiedenen humanen Geweben isolierte Poly-A+-RNA (2 µg) wurde mit einem Formaldehyd-haltigem Agarosegel aufgetrennt, auf eine Nylonmembran geblottet und mit einem markierten FOXG1C-cDNA Fragment (963 - 2218 bp) hybridisiert. Nach dem Waschen wurde die Membran auf einen Film gelegt und nach Belichtung entwickelt. Wie Abbildung 2a entnommen werden kann, wurde im menschlichen Gehim und Hoden eine sehr starke Expression von FOXG1C nachgewiesen, während in Nagern (Ratte und Maus) die Expression von FOXG1C in großem Maße auf das Gehim beschränkt ist. Außerdem ist die Expression von FOXG1C in menschlichen Gehirnabschnitten vermindert oder verstärkt (Abbildung 2b). In Abbildung 2c wurden 10 µg RNA (Bahn 1-3) oder 0,2 µg PolyA⁺ RNA (Bahn 4-6) aus Rattengehirnen auf einem. Gel getrennt, auf eine Membran übertragen und mit einem markierten FOXG1C-cDNA Fragment (963 - 2218 bp) hybridisiert. Die Rattengehime stammten von 3 Wochen alten (Bahn 1 und 4), 6 Wochen alten (Bahn 2 und 5) und 2 Jahren alten Tieren (Bahn 3 und 6). Hierbei wurde eine Altersabhängigkeit der FOXG1C-Genexpression gefunden.

Das aus dem Hefevektor umklonierte FOXG1C cDNA Fragment von 739 bp bis 2218 (AS 175 - 489) wurde mit den Endonukleasen *Eco*RI und *Xba*I in üblicher Weise in den Vektor CMX-Vp16 kloniert und mit MMTV-Luciferase in PC3-ARwt-Zellen, die den AR exprimieren, ebenfalls in üblicher Weise transfiziert.

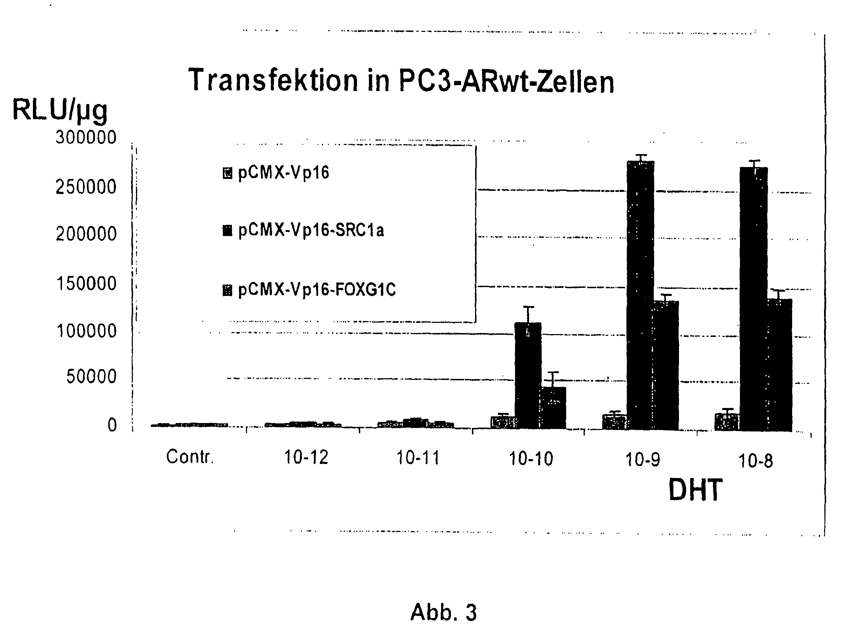

Wie Abbildung 3 entnommen werden kann, führte die transiente Transfektion von FOXG1C-cDNA in frame fusioniert mit der Transaktivierungsdomäne von Vp16 in PC3-ARwt-Zellen zu einer starken Co-Aktivierung der AR-Signaltätigkeit. Dazu wurden in eine Zellkulturschale mit Vertiefungen pro Vertiefung 3x10⁵- Zellen mit 0,43 µg des obigen Konstruktes in CMX-Vp16 bzw. als Negativkontrolle 0,32 µg CMX-Vp16 oder als Positivkontrolle 0,5 µg SRC1a-Vp16 als Kontrollplasmid, 1,0 µg MMTV-Luciferaseplasmid transfiziert und nach 24 Stunden mit Dihydroxytestosteron (DHT) als Androgen in den angegebenen Konzentrationen behandelt. Die transfizierten Zellen wurden nach weiteren 24 Stunden geerntet und die Aktivität des Reportergens Luziferase gemessen. Zusätzlich wurde zur Normierung die Gesamtzellproteinmenge bestimmt. Pro Transfektionsansatz und Substanzkonzentration wurden zwei Experimenten und jeweils drei Messungen durchgeführt. Die Fehlerabweichung wurde als SD angegeben. Die Aktivität ist in relativen Einheiten angegeben.

## Patentansprüche

1. Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon, das die funktionellen Eigenschaften des nukleären Rezeptor-Proteins aufweist, kodiert, und der andere Vektor DNA enthält, die für den Co-Modulator FOXG1C (Genbank XM_007233) oder ein Fragment davon, das die funktionellen Eigenschaften des Co-Modulators aufweist, kodiert, der Substanz ausgesetzt werden und
b) die Transkriptionsaktivität, die der nukleäre Rezeptor oder dessen Fragment, das die funktionellen Eigenschaften des nukleären Rezeptors aufweist, in Anwesenheit des Co-Modulators oder dessen Fragment, das die funktionellen Eigenschaften des Co-Modulators aufweist, auslöst, und/oder der Einfluß der Substanz auf die Interaktion zwischen dem Rezeptor oder dessen Fragment, das die funktionellen Eigenschaften des Rezeptors aufweist, und dem Co-Modulator oder dessen Fragment, das die funktionellen Eigenschaften des Co-Modulators aufweist, durch Protein-Protein-Interaktion oder Protein-Protein-DNA-Interaktion gemessen wird.

2. Verfahren nach Anspruch 1, wobei das Fragment des Co-Modulators, das die funktionellen Eigenschaften des Co-Modulators aufweist, die Aminosäuren 175 bis 489 von FOXG1C (Genbank XM_007233) aufweist.

3. Verfahren nach einem der vorhergehenden Ansprüche, wobei der nukleäre Rezeptor ausgewählt ist unter Androgenrezeptor, Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren.

4. Verfahren nach einem der vorhergehenden Ansprüche, wobei die Zellen etablierte Zelllinien und/oder eukaryotische Zellen sind.

5. Verfahren nach Anspruch 4, wobei die eukaryotischen Zellen unter Prostatazellen, Nervenzellen, Gliazellen, Fibroblasten, Blutzellen, Osteoblasten, Osteoklasten, Hepatozyten, Epithelzellen oder Muskelzellen ausgewählt sind.

6. Verfahren nach einem der vorhergehenden Ansprüche, wobei der Vektor ein eukaryotischer Expressionsvektor ist.

7. Verfahren zur Bestimmung von Störungen im Co-Modulationsmechanismus zwischen Androgenrezeptoren und FOXG1C (Genbank XM_007233), wobei die Konzentrationen von FOXG1C oder einem Fragment davon, das die funktionellen Eigenschaften von FOXG1C aufweist, und/oder Androgenrezeptor oder einem Fragment davon, das die funktionellen Eigenschaften des Androgenrezeptors aufweist, gemessen werden.

8. Verfahren nach Anspruch 7, wobei die Konzentrationsmessung durch Radioimmunassay, ELISA-Tests, Immunfärbung, RT-PCR, Western-Blot, Northem-Blot, DNA-Chip oder Protein-Chip erfolgt.

## Claims

1. Method for testing the hormonal effect, in particular the androgenic or antiandrogenic effect, of substances, said method comprising the steps of:
a) exposing cells transfected with two vectors to the substance, wherein one of the two vectors contains a DNA, which codes for a nuclear receptor protein, or a fragment of said nuclear receptor protein, which has the functional properties of said nuclear receptor protein, and another of the two vectors contains a DNA, which codes for the FOXG1C co-modulator (gene bank XM_007233), or a fragment of said FOXG1C co-modulator, which has the functional properties of said co-modulator, and
b) measuring transcription activity, which said nuclear receptor, or said fragment of said nuclear receptor, which has the functional properties of said nuclear receptor, activates or releases in the presence of said co-modulator or said fragment of said co-modulator, which has the functional properties of said co-modulator, and/or the influence of the substance on an interaction between said receptor, or said fragment of said receptor, which has the functional properties of said receptor, and said co-modulator, or said fragment of said co-modulator, which has the functional properties of said co-modulator, by protein-protein interaction or protein-protein-DNA interaction.

2. Method according to Claim 1, wherein said fragment of said co-modulator, which has the functional properties of the co-modulator has amino acids 175 to 489 of FOXG1C (gene bank XM_007233).

3. Method according to one of the preceding claims, wherein said nuclear receptor is selected from the group consisting of androgen receptor, oestrogen receptor α, oestrogen receptor β, progesterone receptor A, progesterone receptor B, glucocorticoid receptor, mineral corticoid receptor, thyroid gland hormone receptor, vitamin-D receptor, peroxisome proliferator-activated receptor, retinic acid receptor, retinoid X receptor and orphan receptors.

4. Method according to one of the preceding claims, wherein said cells are established cell lines and/or eukaryotic cells.

5. Method according to Claim 4, wherein said eukaryotic cells are selected from the group consisting of prostate cells, nerve cells, glia cells, fibroblast cells, blood cells, osteoblast cells, osteoclast cells, hepatocytes, epithelial cells and muscle cells.

6. Method according to one of the preceding claims, wherein said vector is a eukaryotic expression vector.

7. Method for determining interference in the co-modulation mechanism between androgen receptors and FOXG1C (gene bank XM_007233), said method comprising measuring the concentrations of FOXG1C or a fragment thereof which comprises the functional properties of FOXG1C, and/or androgen receptor or a fragment thereof which comprises the functional properties of the androgen receptor.

8. Method according to Claim 7, wherein said measuring of said concentration and/or said concentrations takes place by radioimmunoassay, ELISA test, immunodyeing, RT-PCR, Western Blot, Northern Blot, DNA chip or protein chip.

## Revendications

1. Procédé de détermination de l'effet hormonal, en particulier de l'effet androgène ou antiandrogène, de substances, dans lequel :
a) des cellules, transfectées par deux vecteurs, le premier vecteur contenant un ADN qui code une protéine de récepteur nucléaire ou un fragment de celle-ci qui présente les propriétés fonctionnelles de la protéine de récepteur nucléaire, et le deuxième vecteur contenant un ADN qui code le co-modulateur FOXG1C (banque génétique XM_007233) ou un fragment de celui-ci qui présente les propriétés fonctionnelles du co-modulateur, sont exposées à la substance, et
b) l'activité de transcription qui, générée par le récepteur nucléaire ou par son fragment, qui présente les propriétés fonctionnelles du récepteur nucléaire, en présence du co-modulateur ou de son fragment, qui présente les propriétés fonctionnelles du co-modulateur, et/ou l'influence de la substance sur l'interaction entre le récepteur ou son fragment, qui présente les propriétés fonctionnelles du récepteur, et le co-modulateur ou son fragment, qui présente les propriétés fonctionnelles du co-modulateur, est mesurée par l'interaction protéine-protéine ou l'interaction protéine-protéine-ADN.

2. Procédé selon la revendication 1, dans lequel le fragment du co-modulateur, qui présente les propriétés fonctionnelles du co-modulateur, présente les acides aminés 175 à 489 du FOXG1C (banque génétique XM_007233).

3. Procédé selon l'une quelconque des revendications précédentes, dans lequel le récepteur nucléaire est choisi parmi un récepteur androgène, un récepteur estrogène α, un récepteur estrogène β, un récepteur de progestérone A, un récepteur de progestérone B, un récepteur de glucocorticoïde, un récepteur minéralocorticoïde, un récepteur hormonal de la glande thyroïdale, un récepteur de la vitamine D, un récepteur activé par le proliférateur de peroxisome, un récepteur d'acide rétinique, un récepteur de rétinoïde X et des récepteurs orphelins.

4. Procédé selon l'une quelconque des revendications précédentes, dans lequel les cellules sont des lignées de cellules établies et/ou des cellules eucaryotiques.

5. Procédé selon la revendication 4, dans lequel les cellules eucaryotiques sont choisies parmi les cellules de la prostate, les cellules nerveuses, les cellules gliales, les fibroblastes, les cellules sanguines, les ostéoblastes, les ostéoclastes, les hépatocytes, les cellules épithéliales ou les cellules musculaires.

6. Procédé selon l'une quelconque des revendications précédentes, dans lequel le vecteur est un vecteur d'expression eucaryotique.

7. Procédé de détermination de troubles dans le mécanisme de co-modulation entre les récepteurs androgènes et le FOXG1C (banque génétique XM_007233), dans lequel les concentrations de FOXG1C ou d'un fragment de celui-ci qui présente les propriétés fonctionnelles du FOXG1C, et/ou d'un récepteur androgène ou d'un fragment de celui-ci, qui présente les propriétés fonctionnelles du récepteur d'androgène, sont mesurées.

8. Procédé selon la revendication 7, dans lequel la mesure de concentration s'effectue par RIA, tests ELISA, coloration immunitaire, RT-PCR, transfert de type western, transfert d'ARN, puce à ADN ou puce à protéine.
